# EUROPEAN PATENT APPLICATION

(11) **EP 2 218 454 A1**
(43) Date of publication of application: **18.08.2010**
(21) Application number: 08829590.2
(22) Date of filing: 05.09.2008
(51) Int. Cl.: A61K 31/405, A61K 31/27, A61K 31/222, A61P 29/00

(54) **PHARMACEUTICAL COMPOSITION COMBINING ACEMETACIN, METHOCARBAMOL AND DIACEREIN, WHICH CAN BE USED IN THE TREATMENT OF RHEUMATOID ARTHRITIS AND RELATED DISEASES**

(30) Priority: 07.09.2007 MX 2007011003
(71) Applicant: World Trade Import-Export, WTIE A.G., 6302 Zug (CH)
(72) Inventor: GARCÍA ARMENTA, María Elena, C.P. 45020 Guadalajara Jalisco (MX); SANTOS MURILLO, Josefina, C.P. 44600 Zapopan Jalisco (MX); ÁLVAREZ OCHOA, Victor Guillermo, C.P. 45222 Zapopan Jalisco (MX)
(74) Representative: Carvajal y Urquijo, Isabel
(86) International application number: PCT/MX2008/000118
(87) International publication number: WO 2009/031877

(57) **Abstract**

The invention relates to a pharmaceutical composition comprising the synergic combination of a nonsteroidal anti-inflammatory known as acemetacin, a centrally acting muscle relaxant known as methocarbamol and a selective inhibitor of interleukin-1 known as diacerein, as well as pharmaceutically acceptable excipients, which are formulated in a single dosage unit to be administered orally. The composition, which is used to control and treat rheumatoid arthritis and related diseases, provides faster pharmacological action and maximal therapeutic effect in less time, as well as reducing inflammation and pain and altering the course of the disease, thereby reducing the risk of side effects.

## Description

### FIELD OF INVENTION

The present invention has application in pharmaceutical industry and discloses a pharmaceutical composition comprising a synergistic combination of a non-steroidal anti-inflammatory agent, such as active principle: Acemetacin; a central action muscle relaxing agent, such as active principle: Methocarbamol and an interleukin-1 selective inhibitor agent, such as active principle: Diacerein; in addition to pharmaceutically acceptable excipients; which are formulated in a single dosage unit to be orally administered, indicated for control and treatment of Rheumatoid arthritis and related diseases.

### BACKGROUND OF INVENTION

Above mentioned active principle combination produces a larger synergistic effect when administered in combination in a single dosage unit unlike when these are independently administered, causing benefits such as: lower concentrations of active principles comprised in the formula, lower administered doses, faster pharmacological action and enhancement of therapeutic effect in less time, in addition to lower risks for side effects to be present.

Rheumatoid arthritis (RA) is a disease which causes joint and surrounding tissue inflammation, causing pain, deformation and motion difficulty, even being capable of affecting other organs or body parts.

It is a chronic disease with a low cure frequency although with a suitable treatment a good disease control is achieved in most cases.

Nuisances and limitations caused by rheumatoid arthritis vary a lot among patients, thus there are not two similar patients. Rheumatoid arthritis is one of the more than 100 existing rheumatoid diseases with specific prognosis and treatment, therefore diagnosis shall be accurate (commonly performed or confirmed by a rheumatologist).

RA is frequent since one out of 200 persons suffers it in our environment. It is a more frequent disease in women but also affecting men. It is not a specific disease of old age and though it may be present on elderly, it is more commonly present in subjects between 45 and 55 years old. Likewise, a very similar form of arthritis may affect children.

Joints are those structures which join bones and which allow human body movement. End bone portions have smooth surfaces which are cartilages, allowing a soft friction between said bones.

In order to nourish and to protect those cartilage-coated bone ends, joints have a membrane (synovial membrane) which coats the inner portion joining one bone with another.

RA is a disease which is caused by synovial membrane inflammation in several joints. This inflammation will cause pain, swelling and a rigidity sensation which may be noticed by morning. Some joints are affected more than others and there are some which are almost never altered. Inflammation persistence in synovial membrane determines that the bone zone gets damaged where synovial membrane is fixed, thus leading to small notches (erosions). Moreover, inflammation maintained by any joint makes that cartilage, which allows a soft friction between bones, is thinned and disappears.

It is possible to achieve that synovial membrane inflammation is controlled with treatment, but damage already produced in bone and cartilages is irreparable. An overload on inflamed joints contributes to accelerate their destruction. In order to get the least possible irreparable damage, the daily reality of patient is indispensable to be known by the physician and that patient participates in treatment by following the required measures.

Although the main location of the lesions caused by RA is in joint synovial membrane, they may often alter other structures.

Rheumatoid nodules are hard lumps (nodules) which may be found in skin appearing in friction zones such as: elbows, finger and toe backside, head back, heel zone, etc. They may also be located within the organism, although they rarely cause relevant lesions for health. These nodules are a consequence of disease activity. Sometimes they spontaneously disappear or when treatment is applied, although they occasionally are removed by surgery.

RA may cause inflammation and atrophy in glands producing tears, saliva, digestive juices or vaginal flow. In this situation, it refers to Sjögren syndrome secondary to RA.

RA may cause inflammation or other type of injury in several organism structures, as well as alterations in blood and urine analysis.

RA is more frequently present in persons with special predisposition; however, it is not a hereditary disease. Cause is unknown. Several infectious agents (bacteria, viruses) have been studied and although suggesting data have been found in some cases, there are no evidences which confirm the involvement of a concrete one.

It is known that there are immune system or body defense alterations: Joint inflammation is a consequence of synovial membrane invasion by immune cells which damage a joint. Defense capability before infections is practically normal in RA patients.

Weather and humidity are not related at all with RA maintenance or triggering. In fact however, some weather changes and particularly when weather tends to worsen makes that any damaged joint whether by this or another disease becomes more painful.

Joint inflammation causes pain and swelling. Joint pain is the most frequent symptom in RA and joint swelling may be more or less visible by patient. Joints which are more frequently inflamed are wrists, knuckles, finger and toe joints, elbows, shoulders, hips, knees and ankles. Neck pain may be also due to rheumatoid arthritis.

In addition to pain and swelling, there may be difficulty in the morning for starting movements (morning rigidity) with variable duration and which may even reach some hours. Persistent inflammation may cause bone, filaments and surrounding tendon damage. The consequence will be a progressive joint deformity and a lack or reduction of joint movement which may lead to patient to a certain impairment degree to perform several common life activities.

Other less frequent symptoms are related with disease alterations in other stages or with less desirable effects for the used treatments. It is more frequent that they are independent such as: uncommon fever, easy tiredness, severe and persistent neck pain, hand or feet tingling, sustained hoarseness without showing a cold, lack of air feeling when under effort, continuous cough, breast or side pain, mouth dryness, redness or sand sensation in eyes, vaginal irritation, skin bumps or persistent diarrhea.

RA diagnosis changes a person's life and that of his/her family, who shall adjust issues often important to their everyday activities because of the new situation.

RA does not currently have a curing treatment; however this does not mean that there is not any treatment at all. New drugs are appearing which allow controlling the disease in an increasingly higher percentage in patients.

In addition to the general measures for joint care, pharmacological treatment shall be started. All drugs have secondary effects and also those used for RA treatment.

RA treatment with drugs includes two groups of drugs. One of them includes those intended for releasing pain and inflammation in short term but which are not useful for modifying long-term disease evolution. Non-steroidal antiinflammatories (NSAIDs) and glucocorticoids are within this group.

Non-steroidal antiinflammatories are efficient drugs. Glucocorticoids are judiciously used, at moderate dosages for specific conditions, allowing in many cases an improvement in long-term patient life quality, overcoming the side effects caused with these dosages.

The other large group is that of the disease modifying drugs. These drugs act by making long-term disease activity to become lower. They make effect along weeks or even months. They are not efficient in 100% of patients, therefore being common to prescribe them sequentially in order to find that which is more efficient and better tolerated. Within this group are: Methotrexate, sulfazalazine, gold salts, chloroquine, cyclosporine, D-penicillamine, azatrioprine, etc.

On the other hand, the new drugs which modify the disease include multiple drugs which act at TNF level (tumor necrosis factor); this factor is acknowledged as a RA pathogenic molecule since by blocking TNF in patients with RA, it has demonstrated to significantly delay joint damage. TNF human over-expression develops synovitis in experimental animals together with bone and cartilage structure destruction.

RA is a chronic polyarthritis which leads to bone destruction and severe joint dysfunction.

However, and in spite of having a number of drugs currently available which are administered to patients suffering from RA, the treatment prescribed for said patients is not fully effective in most cases, which in addition to manifesting side effects frequently limits its long-term uses. Classical non-steroidal antiinflammatory drugs are used for RA symptom control, but they are associated with a significant gastrointestinal toxicity, including a potential risk of gastroduodenal perforations, ulcers and bleeding. RA therapy with slow-action antirrheumatic disease modifying drugs such as Methotrexate, is generally accepted as a long term standard treatment, since it provides a significant symptom reduction, but does not arrest cartilage destruction. Glucocorticoids, the most powerful and significant immunosuppresants, are used for acute and severe joint inflammation but they are not used in chronic therapy in most patients since they show significant side effects.

The new therapeutic agents such as: monoclonal antibodies, human receptor immunoglobulin constructs, cytokines or human recombinant proteins have been proved in RA and in other chronic arthritic diseases such as ankylosing spondilitis or psoriatic arthritis, with a convincing success evidence. Particularly, the clinical studies assessing anti-TNF agents, whether alone or in combination with methotrexate, have provided feasibility and efficacy in these new approaches. However, the therapy which is directed to TNF and IL-1 inhibition is clinically effective in only 40 to 70% from patients, and these are significantly the TNF antagonist therapies which are associated with side effects including: tuberculosis, lysteriosis, lymphomas and hystoplasmosis. Figures are not so high but a clinical observation is necessary for lowering risks.

Treatments which are directed against osteoclasts, such as osteoprotegerin, show promising for bone destruction prevention in RA experimental models, but this therapy does not provide advancements as to inflammatory tissue or as to disease symptoms.

Most of drugs which are currently available in marketplace for rheumatoid arthritis treatment comprise principles which are independently formulated and fulfilling with a specific therapeutic activity; however these drugs, specifically disease modificators, show a large amount of side effects which consequently make patients to cancel their treatment, thus causing an increase in symptomatology and a manifestation of severe complications, both by treatment cancellation and for a continuation thereof; for instance, the use of Methotrexate which although well-tolerated in some patients which already have a good response to treatment, is fully non-tolerated in other patients because of the caused side effects.

Rheumatoid arthritis is a disease with a very wide and varied spectrum including from the mild disease forms which deserve a limited treatment and which are compatible with a fully normal life, up to the most severe forms thereof which may lead to shorten the patient's life expectancy, especially when other pathologies are present in addition to said disease, which makes the already existent clinical condition even more complicated.

In any case, when disease is left to an evolution without treatment, it will have a bad prognosis and finally leads to a significant functional impairment in affected joints.

### SUMMARY OF INVENTION

In order to provide a pharmaceutical alternative which achieves a reduction in patients symptomatology when suffering RA, such as inflammation, pain and disease modification effects, in addition to decrease the risk that secondary effects are present, the present invention development was carried out, whereby a pharmaceutical composition is described comprising a combination of a non-steroidal anti-inflammatory agent, a central action muscle relaxing agent and an interleukin-1 selective inhibitor agent, which together act synergistically and which are formulated in a single dosage unit to be orally administered, which provides significant benefits such as: lower concentrations of active principles comprised in the formula, lower administered doses, enhancement of therapeutic effect and faster pharmacological action in a shorter time, as well as a modification of disease course and a reduction on risks which manifest severe complications.

### DETAILED DESCRIPTION OF INVENTION

Non-steroidal antiinflammatories (NSAIDs) agents include a large group of drugs which have an important analgesic, antiinflammatory and antipyretic activity, in addition to other therapeutic effects.

A classification is available for these drugs:

### ACIDS:

a) Salicylates (acetylsalicylic acid); b) Enolic: Pyrazolones (Metamizole), Pyrazolidinediones (Phenylbutazone), Oxicams (Piroxicam and Meloxicam); c) Acetic: Indolacetic (Indometacin, Acemetacin), Pyrrolacetic (Ketorolac), Phenylacetic (Diclofenac), Pyranoindoacetic (Etodolac); d) Propionic (Naproxene, Ibuprofen); e) Antranilic (mephenamic acid); f ) Nicotinic (Clonixine).

### NON-ACIDS:

a) Sulfoanilides (Nimesulide); b) Alcanones (Nabumetone); c) Paraaminophenols (Paracetamol).

### COXIB: (COX-2 selective inhibitors)

a) Sulfonamides: Celecoxib, Parecoxib, Lumiracoxib, Etoricoxib, Rofecoxib (removed) and Valdecoxib (removed).

Non-steroidal antiinflammatories show their analgesic action by relieving pain associated with inflammation or with a tissue lesion by decreasing prostanoid production which sensibilize nociceptors to mediators, such as bradykinin. They are efficient in mild to moderate intensity pains. Their antipyretic effect is shown by prostaglandin production inhibition in hypothalamus and interference with temperature regulating mechanisms.

Their anti-inflammatory activity is produced by reducing the components of the inflammatory response wherein COX products perform a significant role such as: vasodilatation, edema and pain.

All non-steroidal antiinflammatories are analgesic and antipyretic, some (indometacin, piroxicam) are more antiinflammatory, most are moderately antiinflammatory (ibuprofen, nabumetone) and others (paracetamol) have a minimum anti-inflammatory effect.

Some non-steroidal antiinflammatories have platelet antiaggregating activity, which is of special interest in the case of the acetylsalicylic acid because of its platelet COX irreversible inhibiting effect. This NSAID is of great use in prevention of coronary and brain thromboembolic accidents. Similarly, some NSAIDs have uricosuric action as a consequence of uric acid transport inhibition from renal tubule lumen to interstitial space. It is only appreciated with some NSAIDs to high doses, such as phenylbutazone, sulfinpyrazone or salicylates.

Under NSAID action mechanisms, all their effects are related with cyclooxygenase inhibition (COX) and with prostaglandin production inhibition. AAS is the only which produces a COX-1 irreversible inhibition. NSAID antiinflammatory effect is clearly related with COX-2 inhibition and many of the undesirable effects are related with COX-1 inhibition.

Acemetacin development was carried out from a systematic alteration of indometacin molecule through changes in α-methyl group from indol-3-acetic acid, in carboxyl group and substitution of the basic indole structure with supplementary heterocyclic rings.

A combination of compound number 54 was found within the series of indometacin esters, which in rat leg kaolin edema model displays an anti-inflammatory action about 2-fold higher. After several assays the acemetacin molecule was synthesized, which chemical formula is as follows: [1- (p-chlorbenzoyl)-5-methoxy-2-methyl-indol-3-acetoxyl acetic acid. Acemetacin (AC) is classified as an indometacin carboxy methyl ester derived non-steroidal antiinflammatory. Its pharmacological properties are determined through studies performed in animal models (mouse, rat, guinea pigs, cat, rabbit, dog). In humans (healthy or sick volunteers): in isolated organs, phetus, healthy or inflammated tissues, through thin coat chromatography, ultraviolet spectroscopy and fluorescence.

Acemetacin fixation to human albumina depends on protein concentration oscillating between 81.5 and 93.7%, with a free combination availability fraction of about 60%, which suggests that Acemetacin effect manifestation is faster.

Renal clearance for more conjugated free indometacin and that for Acemetacin was performed at 29.9 mL./min. (9 hours later) and 32.6 mL./min. (24 hours later), without keeping a relationship with administration route. Mean terminal clearance half-period for Acemetacin is determined in 5.84 hours. This clearance suggests that Acemetacin accumulation risk is not significant.

Acemetacin is a prostaglandin synthesis weak inhibitor, preferably affecting COX-2 and to COX-1 in a lower degree, thus conferring the property of prostaglandin synthesis inhibition related with inflammatory reaction, but affecting very little those related with cellular physiology; moreover, it inhibits serotonin, bradykinin and histamine, which are known inflammation mediators. COX-2 potent inhibition and COX-1 weak inhibition make Acemetacin to have a low renal and gastric toxicity profile, but a potent antiinflammatory effect.

Acemetacin has partially a prostaglandin inhibition effect somehow dose-dependent, mainly affecting to PGE2, being necessary that Acemetacin concentration which inhibits said synthesis is higher, which reflects a lower potency for Acemetacin to inhibit prostaglandins, this being related with a low lesion incidence in gastric mucose.

Central action muscle relaxing agents act over internuncial neurons suppressing polysynaptic ways, exerting its action over higher autonomous nerve centers. They are also used as tranquilizing agents.

Muscle contracture (CM) is manifested when a sudden, violent and involuntary muscle tension increase, as a result of traumas, inflammatory type alterations or other type of lesions in skeletal muscle. This is a more or less steady state which produces pain both in rest and motion conditions, thus limiting the performance of joints related with muscle or affected muscle group.

Muscle contracture involves a nociceptive afferent conduction from damaged tissue, stimulated by alpha-motor neurons up to muscle, leading to an affected muscle painful tonic contracture, creating a vicious circle since caused pain by spasm causes a larger nociceptive stimulus which upon exciting the alpha-mtor neurons, causes more spasm and more pain.

Descendent way dysfunction (corticospinal, vestibulospinal, reticuloepinal) which exerts control over alpha-motor neurons is also included in muscle contracture physiopathology. Peripheral reflex arcs although they are shown hyperactive due to an abnormal control received from upper nervous centers, they apparently do not intervene in pathologic process; however, some sites where these arcs have influence, including afferent nervous fibers from skin and muscle spindles, spinal cord interneurons, efferent nervous fibers and intrafusal and extrafusal muscle fibers use to be blanks for pharmacological intervention which shall control an increase in muscle tone.

Physical and sport activity causes a number of situations which lead to locomotive apparatus pain. Besides the circumstances due to joint and bone lesions, the presence of local pains in muscle mass occupies a remarkable place within the main causes of medical visit.

Myalgia is very vague and therefore is referred to as cramps, contractures, spasms and a number of terms which eventually do not allow a definition of specific situations. In other times, the origin of lesion allows more specificity and it refers to fibrillar break or bruise, upon identifying a specific trauma as a cause of muscle pain.

Physiological mechanisms of muscle-tendon proper contracture and innervations explain pain physiopathology: defective relaxation, change in tone control by gamma loop, participation of alpha-motor neurons and nociceptive innervation, in addition to the significant role performed by microcirculation.

Muscle processes related with pain show an intimate inflammation substrate or local interstitial edema. The fact that an increase in tissue or contracture stress is frequently noticed makes that medical treatment under these situations requires generally the use of muscle relaxants together with analgesics or antiinflammatories.

Sport practice involves several situations of muscular pain due to traumatic, microtraumatic or overload insults. These several causes comprise from acute traumatism which injures because of its intensity, until a small stress injuring because the muscle is tired or overloaded. In all cases, pain is spontaneously present on palpation and with muscle function. Skeletal muscle relaxing drugs are used for treatment of pathological processes associated with muscle contractures and having the main purpose of restoring normal muscle excitability without deeply affecting motor function. Muscle relaxants shall not prevent contracture but restoring a normal excitability and muscle tone to decrease pain and to improve motor function.

Methocarbamol is a mephenesin derived analog carbamate which produces polysynaptic reflex inhibition, which is performed as central action muscle relaxant.

Methocarbamol pharmacological action start occurs within the first 30 minutes after being orally administered, reaching its maximum effect 3 to 4 hours after administration, with a total therapeutic action duration of about 24 hours. Its half life is approximately 14 hours.

Methocarbamol is absorbed almost completely after being orally administered; it is metabolized through dealkylation and hydroxylation and its clearance is performed mainly through urine as glucuronide and metabolite sulfate conjugates, and only a small amount thereof is excreted through feces.

Methocarbamol action mechanism has not yet been well established; however, research results conducted on animals have demonstrated that it acts by reducing the polysynaptic reflex activity in spine cord, and this mechanism achieves a reduction in alpha-motor neuron excitability, thus providing a skeletal muscle relaxation; moreover it has a depressive effect over central nervous system, leading to a sedative state which is mainly related with the muscle relaxant effect.

It is used as adjuvant therapy in treatment of symptoms manifested by painful muscle spasm disorders.

Usual dose used for orally administered Methocarbamol is up to 1.5 g four times a day, then reducing to a maintenance dose of 4 g per day along 2 to 3 days. A 750 mg dose, three times a day has been determined to be sufficient for providing an efficient therapeutic effect.

Methocarbamol may be also intramusculary administered with a 500 mg dose every 8 hours; intravenously at a dose of 300 mg per minute in slow injection or by infusion in sodium chloride or glucose. Parenteral dose shall not exceed 3 g daily during 3 days.

Interleukin-1 selective inhibitor agents act by blocking Interleukin-1 activity which is produced by monocytes and macrophages as an early response from immune system, stimulating T cell proliferation and protein synthesis.

Body immune system which usually provides protection against harmful foreign influences may mistakenly react. Sometimes immune system does not recognize body tissues as own and destroys them causing disease. Among theses "autoimmune diseases" rheumatoid arthritis is classified.

Cytokines are messenger molecules whereby cells communicate each other. In rheumatoid arthritis, defective immune system produces cytokines in excess which favors inflammation. One of the key cytokines in the process of rheumatoid arthritis appearance is a cytokine named Interleukin-1 (IL-I). Interleukin-1 is released by emitting cells and is bound to specific receptors. This attachment produces a number of reactions. Immune system induces IL-I formation in synovial membrane; being there where Interleukin-1 causes cartilage and bone inflammation and destruction.

Interleukin-1 is a glicoproteinaceous nature molecule which is found in two biological active forms, IL-1 alpha (IL-1α) and IL-lbeta (IL-1β). Both interleukins are bound to the same receptor, in spite of not sharing a large homology in aminoacid sequence (22%). There is another molecule similar to IL-1beta which is bound to the same receptor, but unlike the first two, this does not manifest any biological activity, being named as IL-1 receptor antagonist (IL-1Ra) because of this feature.

The three molecules constitute the IL-1 family. Genes encoding each of the members of this family are located in chromosome 2 long arm, site where genes for the two receptor types which attach to IL-1 are also located.

Immune system produces opposite molecules called antagonists, which are attached to the receptor impeding Interleukin-1 attachment. In a healthy joint, there is sufficient amount of antagonists which control inflammatory process. In rheumatoid arthritis there is an imbalance between Interleukin-1 and its antagonist, because there is a very high amount of Interleukin-1 within the joint and a very little amount of antagonist. Consequently, Interleukin-1 occupies disproportionately many receptor sites, thus maintaining the destructive and inflammatory process in joints.

Diacerein is a purified compound with anthraquinone structure, having inhibiting activity in vitro and in vivo over Interleukin-1 production and activity and metalloprotease secretion without altering prostaglandin synthesis, providing a remodeling mechanism in joint cartilage.

Diacerein has demonstrated to be effective in treatment of osteoarthrosis (OA), a degenerative joint process characterized in a progressive cartilage destruction and erosion. The use of Diacerein in OA animal models, as well as poliarthritis models (NZB/KN male mice), revealed that this consistently moderates cartilage degradation, preventing or reducing micro and macroscopic lesions in joint tissue. Diacerein oral administration to patients with hip OA was associated with a symptomatic improvement and a modifying effect of cartilage structure, together with a good safety profile.

Interleukin-1 is a factor which intervenes in macrophage-neutrophil response to inflammation and performs an important role in joint cartilage degradation. This factor together with TNF (Tumor Necrosis Factor) and other colony stimulating factors produce a retroaction mechanism which starts with tissue inflammation, followed by leukocyte formation.

Several studies have demonstrated that Diacerein: Inhibits interleukin-1 production and decreases the collagenolytic activity.

Diacerein antiarthrosic properties are due to its capability of inhibiting pro-inflammatory and pro-catabolic cytokines, such as Interleukin-1, which performs a significant role in joint cartilage degradation, as well as inhibition of enzyme production and release which degrade cartilage (collagenase and stromelysin).

After being orally administered, Diacerein is hydrolized before penetrating into systemic circulation and suffers a first stage liver metabolism, being fully deacetylated and becoming into a rein form and its conjugates. Simultaneous intake of Diacerein with food causes a delay in absorption by extending tmax and also increasing bioavailability. Rein is attached to plasma proteins in 99%. Its excretion is by renal route as rein and its conjugates (glucuronides and sulfates). Diacerein clearance half-life in plasma is from 5 to 7 hours.

Diacerein has a slow action start which is not significant until 4 weeks after treatment. The effect is maintained at least during 2 months after treatment has been cancelled.

Nowadays, we may find in marketplace a number of pharmaceutical products which are directed to rheumatoid arthritis treatment comprising several active principles being independently formulated; however, in this kind of diseases is very important to prevent or to reduce the appearance of inflammatory and painful processes caused by said pathology, as well as achieving a modification in disease course; consequently, the activity of a drug group acting through different mechanisms is indispensable to remove or to decrease inflammation, pain, and to modify the disease course, further reducing the risk of manifesting secondary effects.

In the light of the above and in order to provide a useful pharmacological option for control and treatment of rheumatoid arthritis and other related pathologies, the development of present pharmaceutical composition was carried out, which comprises a synergistic combination of a non-steroidal anti-inflammatory agent, such as active principle: Acemetacin, a central action muscle relaxing agent, such as active principle: Methocarbamol and an interleukin-1 selective inhibitor agent, such as active principle: Diacerein, in addition to pharmaceutically acceptable excipients; which are formulated in a single dosage unit to be orally administered .

Said formulation has been developed considering that active principles comprised in the pharmaceutical composition subject of the present invention have a significant power and efficacy for preventing or reducing the presence of inflammatory and/or painful processes which usually are together with said disease, in addition to modify the course thereof by preventing its progression, and also providing a faster pharmacological action and a maximization of its therapeutic effect in a shorter time, as well as a decrease in the risks that secondary effects are manifested.

The non-steroidal anti-inflammatory agent used in the pharmaceutical composition subject of the present invention, such as active principle: Acemetacin, is present in the formulation in a concentration range from 30.0 mg to 180.0 mg per dose unit.

The central action muscle relaxing agent used in the pharmaceutical composition subject of the present invention, such as active principle: Methocarbamol, is present in the formulation in a concentration range from 100.0 mg to 850.0 mg per dose unit.

The interleukin-1 selective inhibitor agent used in the pharmaceutical composition subject of the present invention, such as active principle: Diacerein, is present in the formulation in a concentration range from 25.0 mg to 100.0 mg per dose unit.

In order to assess the pharmaceutical composition subject of the present invention, as well as the synergistic effect resulting from a combination of active principles: Acemetacin, Methocarbamol and Diacerein in a single dosage unit, a comparative clinical study was carried out wherein above mentioned active principles were separately administered, as well as a combination thereof.

### ACEMETACIN / METHOCARBAMOL / DIACEREIN VS. ACEMETACIN / METHOCARBAMOL / METHOTREXATE COMPARATIVE CLINICAL STUDY FOR TREATMENT OF RHEUMATOID ARTHRITIS.

We conducted a clinical study on 50 patients with rheumatoid arthritis diagnosis. The study was comparative, prospective, open.

50 patients were divided into two treatment groups:
- Group 1: received 90 mg Acemetacin treatment every 12 hours, 215 mg Methocarbamol every 12 hours and 2.5 mg Methotrexate every other day.
- Group 2: received Acemetacin / Methocarbamol / Diacerein treatment twice a day, during 6 follow-up weeks.

A full clinical report was prepared as well as synovitis, joint degradation and bone erosion assessment to each one of the patients.

### RESULTS.

Forty three patients completed the study; seven patients from Group 1 stopped treatment because of side effects on 3rd follow-up week, side effects were nausea and vomit, see Table 1.

**Table 1. Baseline demographic data**

| **Patients** | **Group 1** | **Group 2** |
|---|---|---|
| | **(n=18)** | **(n=25)** |
| Sex F/M | 14/4 | 20/5 |
| Age | 43±6 | 45 ± 5 |
| Rheumatoid Arthritis | 100% | 100% |

Upon study completion patients from Group 1 and Group 2 showed a disease improvement (Rheumatoid arthritis), which was 100% for both groups. Upon study completion, among patients treated with Acemetacin / Methocarbamol / Methotrexate, 50% of them (n=9) showed mild to severe alterations compared with Group 2, which received Acemetacin / Methocarbamol / Diacerein, wherein only 11% from total patients treated with this combination (n=3) showed moderate to severe alterations, see Table 2.

**Table 2. Data obtained at six weeks of treatment**

| | **Group 1** | **Group 2** |
|---|---|---|
| | **(n=18)** | **(n=25)** |
| Moderate to severe intensity arthritis | 9 / 50% | 3 / 11% |

### CONCLUSIONS

Non-steroidal antiinflammatory drugs are used for RA symptom control; therapy with slow action antirrheumatic disease modifying drugs, such as Methotrexate, which is accepted as a long term standard treatment, has a symptom reduction but does not arrest cartilage destruction. Methocarbamol is used as a coadjuvant for prevention and/or treatment of muscle contracture. Administration of this combination with Acemetacin and Diacerein, which are well-tolerated therapeutic agents, clearly demonstrates that said composition has a significant antiarthritic activity, preventing from the beginning of disease a manifestation of painful and inflammatory processes which are associated with Rheumatoid arthritis, in addition to cause a suppression of joint pathology progression.

Preservation of joint architecture after Acemetacin + Methocarbamol + Diacerein combination administration is mainly due to a suppression of synovial tissue high proliferation, also called pannus, which comprises myleomonocytic type synoviocytes, such as monocytes and synovial macrophages, and mesenchymal type synoviocytes, such as synovial fibroblasts, in addition to several infiltrating inflammatory cells which contribute to joint bone and cartilage invasion and destruction.

## Claims

1. A pharmaceutical composition **characterized in that** comprises a synergistic combination of a non-steroidal anti-inflammatory agent, such as active principle: Acemetacin, a central action muscle relaxing agent, such as active principle: Methocarbamol and an interleukin-1. selective inhibitor agent, such as active principle: Diacerein, in addition to pharmaceutically acceptable excipients; wherein active principles are present in formulation in a concentration range from 30.0 mg to 180.0 mg for Acemetacin, from 100.0 mg to 850.0 mg for Methocarbamol and from 25.0 mg to 100.0 mg for Diacerein; those which are formulated in a single dosage unit to be orally administered, which is indicated for control and treatment of rheumatoid arthritis and related pathologies.

2. A pharmaceutical composition according to claim 1, **characterized in that** the non-steroidal anti-inflammatory agent, such as active principle: Acemetacin, is present in the formulation in a concentration range from 30.0 mg to 180.0 mg, being preferably used a concentration formulation of 90.0 mg per dose unit.

3. A pharmaceutical composition according to claim 1 and 2, **characterized in that** the central action muscle relaxing agent., such as active principle: Methocarbamol, is present in the formulation in a concentration range from 100.0 mg to 850.0 mg, being preferably used a concentration formulation of 215.0 mg per dose unit.

4. A pharmaceutical composition according to claims 1 to 3, **characterized in that** interleukin-1 selective inhibitor agent, such as active principle: Diacerein, is present in the formulation in a concentration range from 25.0 mg to 100.0 mg, being preferably used a concentration formulation of 50.0 mg per dose unit.

5. A pharmaceutical composition according to claims 1 to 4, **characterized in that** is formulated in a single dosage unit to be orally administered in capsule or tablet form.

6. The use of the pharmaceutical composition according to claims 1 to 5, **characterized in that** is indicated for control and treatment of rheumatoid arthritis and related diseases.

7. A pharmaceutical composition according to claims 1 to 6, **characterized in that** manifests a significant potency and efficacy for preventing or reducing the presence of inflammatory and/or painful processes which usually go with rheumatoid arthritis and modifying the course thereof by preventing its progression, in addition to provide a faster pharmacological action and enhancement of therapeutic effect in shorter time, also decreasing the risks of showing severe side effects or complications.
